(19) Europäisches Patentamt
European Patent Office
Office européen des brevets

(11) Veröffentlichungsnummer: **0 082 413**
**B1**

(12) **EUROPÄISCHE PATENTSCHRIFT**

(45) Veröffentlichungstag der Patentschrift: **24.07.85**

(51) Int. Cl.⁴: **C 07 C 69/712**, C 07 C 67/31

(21) Anmeldenummer: **82111366.9**

(22) Anmeldetag: **08.12.82**

(54) **Verfahren zur Herstellung von 2-(Hydroxyphenoxy)-carbonsäureestern.**

(30) Priorität: **18.12.81 DE 3150233**

(43) Veröffentlichungstag der Anmeldung: **29.06.83 Patentblatt 83/26**

(45) Bekanntmachung des Hinweises auf die Patenterteilung: **24.07.85 Patentblatt 85/30**

(84) Benannte Vertragsstaaten: **BE DE FR GB IT**

(56) Entgegenhaltungen:
**US - A - 3 709 939**
**US - A - 3 860 633**

(73) Patentinhaber: **BASF Aktiengesellschaft, Carl-Bosch-Strasse 38, D-6700 Ludwigshafen (DE)**

(72) Erfinder: **Becker, Rainer, Dr., Sonnenwendstrasse 83, D-6702 Bad Duerkheim (DE)**
Erfinder: **Oeser, Heinz-Guenter, Dr., Mohngasse 25, D-6716 Dirmstein (DE)**
Erfinder: **Acker, Rolf-Dieter, Dr., Tuchbleiche 8, D-6906 Leimen (DE)**

EP 0 082 413 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von 2-(Hydroxiphenoxi)-carbonsäureestern, die teilweise bekannt sind. Diese Verbindungen sind als Zwischenprodukte für die Synthese biologisch, insbesondere herbizid aktiver Wirkstoffe wertvoll.

Es ist bekannt, dass die gezielte Monoalkylierung von Dihydroxibenzolen Schwierigkeiten bereitet, da die Neigung besteht, dass beide Hydroxylgruppen mit dem Alkylierungsmittel reagieren. Das gilt zwar nicht für asymmetrisch dihalogenierte Hydrochinone, wie aus US-A-3709939 und US-A-3860633 hervorgeht, wohl aber für unsubstituierte. So beschreiben Newman und Cella, J. Org. Chem. *39* (1974), 214 f., u.a. die Alkylierung von Hydrochinon in Form seines Dinatriumsalzes mit 2-Bromcarbonsäureestern in gewissen wasserlöslichen organischen Lösungsmitteln. Es hat sich aber gezeigt, dass diese Methode nicht allgemein anwendbar ist. Das aus der DE-OS-2824828 bekannte Verfahren, bei dem Hydrochinon mit 2-Halogencarbonsäureestern in alkoholischer Lösung in Gegenwart von Natriumalkoholat umgesetzt wird, ist nur auf Ester mit niedermolekularer Estergruppe anwendbar, bei den Butylestern versagt es bereits; auch geht bei diesem Verfahren eine beim Ausgangsester vorhandene optische Aktivität verloren. Schliesslich ist aus einer Arbeit von Moser, J. Amer. Chem. Soc. 72 (1950), 1413 ff., die Umsetzung von Hydrochinon mit Chloressigsäureethylester in Gegenwart von Borfluorid bekannt; bei dieser Methode tritt nebeneinander Mono- und Disubstitution des Hydrochinons ein.

Es wurde nun gefunden, dass man die genannten Schwierigkeiten vermeiden und auf vorteilhafte Weise 2-(Hydroxiphenoxi)-carbonsäureester der allgemeinen Formel

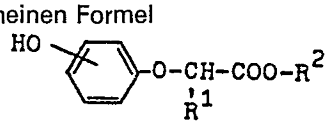

I,

in der $R^1$ Wasserstoff oder eine niedermolekulare Alkylgruppe, vorzugsweise eine Methyl- oder eine Ethylgruppe und $R^2$ einen gesättigten oder ungesättigten Kohlenwasserstoffrest mit bis zu 10 Kohlenstoffatomen, der gegebenenfalls mit Phenoxy- oder Cyangruppen, Halogen oder Trialkylsilyl substituiert ist, oder einen Rest der Formel

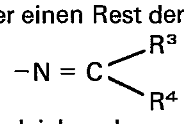

und $R^3$ und $R^4$ gleiche oder verschiedene Kohlenwasserstoffreste mit zusammen bis zu 9 Kohlenstoffatomen, die auch miteinander verbunden sein können, bedeuten, herstellen kann, wenn man eine Verbindung der Formel

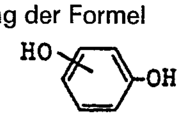

II

mit einem 2-Halogenfettsäureester der Formel

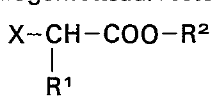

III

in der $R^1$ und $R^2$ die oben angegebene Bedeutung haben und X ein Chlor- oder Bromatom bedeutet, in Gegenwart von Calciumhydroxid in Dimethylsulfoxid bei einer Temperatur zwischen 0 und +100°C umsetzt, wobei das Molverhältnis der Ausgangsstoffe II:III zu Beginn der Umsetzung wenigstens 1,5:1 beträgt.

Bei den Ausgangsstoffen der Formel II handelt es sich um Hydrochinon, Resorcin und Brenzkatechin. Bevorzugt sind die beiden erstgenannten. Die Ausgangsstoffe der Formel III sind bekannt oder können nach bekannten Verfahren erhalten werden, beispielsweise durch die übliche Veresterung eines Alkohols, Phenols oder Oxims der Formel $HO-R^2$ mit einem Chlorid oder Anhydrid der Chlor- oder Bromessigsäure oder höherer Homologen dieser Säuren, wie vor allem 2-Chlor- oder 2-Brompropion- oder -buttersäure. $R^2$ hat dabei die oben angegebene Bedeutung und kann insbesondere ein bis zu 10, vorzugsweise bis zu 8 Kohlenstoffatome enthaltender geradkettiger oder verzweigter Alkyl-, Alkenyl-, Alkinyl-, Cycloalkyl, Aryl-, Aralkylrest oder Rest eines offenkettigen oder ringförmigen Ketoxims sein. Der Rest $R^2$ kann substituiert sein, wobei im Hinblick auf die Wirksamkeit der Endprodukte für die die erfindungsgemäss erhältlichen Stoffe Zwischenprodukte darstellen, als Substituenten insbesondere Phenoxigruppen, Cyangruppen, Halogenatome, wie vor allem Brom und Chlor, und Trialkylsilylgruppen in Betracht kommen.

Als Beispiele für Ausgangsstoffe der Formel III seien genannt: Bromessigsäuremethylester, Chloressigsäureethylester, 2-Chlor- und 2-Brompropionsäure-methyl-, -ethyl-, -n-propyl-, -2-phenoxiethyl-, -3-phenoxipropyl-, -n-butyl-, -isobutyl-, -t-butyl-, -n-pentyl-, -n-octyl-, -2-ethylhexyl-, -n-decyl-, -allyl-, -propargyl-, -2-cyanethyl-, -1-cyanpropyl-, -3-chlorallyl-, -2,3-dichlorallyl-, -2-bromallyl- und -phenylester, 2-Brombuttersäureethylester, 2-Brombuttersäure-1-cyancyclohexylester, 2-Bromvaleriansäureethylester, 2-Bromcapronsäuremethylester, 2-Brompropionyl-acetonoxim und Bromacetyl-cyclohexanonoxim.

Diejenigen Stoffe der Formel III, in denen $R^1$ Alkyl ist, besitzen ein asymmetrisch substituiertes Kohlenstoffatom. Sie können daher als Racemat oder in optisch aktiver Form vorliegen. Es ist ein besonderer Vorteil des erfindungsgemässen Verfahrens, dass bei der Verwendung eines optisch aktiven Ausgangsstoffs der Formel III die optische Aktivität im Verfahrensprodukt der Formel I erhalten bleibt.

Das erfindungsgemässe Verfahren wird in organischer Lösung durchgeführt. Von den vielen organischen Lösungsmitteln, die grundsätzlich in Betracht zu ziehen wären, hat vor allem eines alle Anforderungen in bezug auf Selektivität der Monosubstitution, Ausbeute und breite Anwendbarkeit voll befriedigen können, nämlich das Dimethylsulfoxid.

Für den Erfolg des erfindungsgemässen Verfahrens ist das Moverhältnis, in dem die Ausgangsstoffe eingesetzt werden, nicht ohne Bedeutung. So

sollen je Mol Ausgangsstoff der Formel III wenigstens 1,5 Mol Verbindung der Formel II eingesetzt werden. Vorzugsweise wählt man das Molverhältnis II:III etwa 2:1. Man kann auch über dieses Verhältnis noch hinausgehen, doch bringt das in der Regel keine Vorteile. Das Calciumhydroxid setzt man im allgemeinen in etwa der halben molaren Menge, bezogen auf die Verbindung der Formel II, ein. Ein Abweichen von diesem Verhältnis bringt in der Regel keine Vorteile und es empfiehlt sich gewöhnlich nicht, weniger als 0,4 Mol oder mehr als 1,0 Mol Calciumhydroxid, bezogen auf ein Mol der Verbindung der Formel II, zu verwenden.

Die Reaktionstemperatur liegt bei dem erfindungsgemässen Verfahren zwischen 0 und +100°C. Besonders vorteilhaft arbeitet man meist im Bereich von 0 bis 60°C. Die günstigste Reaktionstemperatur ist bis zu einem gewissen Grad von der Wahl der Reaktionspartner abhängig. So reagieren von den Stoffen der Formel III die 2-Bromfettsäureester bei niedrigeren Temperaturen als ihre Chloranaloga. Für die ersteren hat sich daher ein Temperaturbereich von ungefähr 0 bis 20°C besonders bewährt, wobei die untere Grenze durch die Gefahr, dass das Reaktionsmedium erstarrt, bedingt ist, während die Gefahr merklicher Disubstitution der Stoffe der Formel II umso mehr wächst, je mehr man den genannten Bereich überschreitet. Eine Reaktionstemperatur um 10°C bietet bei Einsatz von 2-Bromfettsäureestern grösstmögliche Sicherheit. Setzt man dagegen 2-Chlorfettsäureester der Formel III ein, so liegt der bevorzugte Temperaturbereich höher, und zwar im allgemeinen bei etwa 25 bis 90°C; besonders vorteilhafte Ergebnisse erzielt man in diesem Fall bei einer Reaktionstemperatur um 50°C. Hat man bei der Durchführung des erfindungsgemässen Verfahrens die Wahl zwischen einem bestimmten 2-Bromettsäureester und seinem Chloranalogon als Ausgangsstoff, so ist im allgemeinen der Einsatz der Bromverbindung vorzuziehen, da diese meist höhere Ausbeuten an dem gewünschten Verfahrensprodukt liefert.

Eine vorteilhafte Art, das erfindungsgemässe Verfahren durchzuführen, besteht darin, den Ausgangsstoff der Formel II in Dimethylsulfoxid zu lösen, in dieser Lösung das fein verteilte Calciumhydroxid zu dispergieren und zu dem Ansatz unter Einhaltung der gewünschten Reaktionstemperatur den Ausgangsstoff der Formel III langsam zuzugeben. Das Lösungsmittel sollte zweckmässigerweise nur geringe Mengen, beispielsweise weniger als 1%, an Wasser enthalten. Die Menge an Lösungsmittel kann in einem weiten Bereich gewählt werden. Bewährt haben sich beispielsweise Mengen von 0,2 bis 1 l/Mol der Verbindung II. Die Umsetzung ist in der Regel in einigen Stunden beendet. Die Isolierung des Verfahrensprodukts erfolgt nach üblichen Methoden, beispielsweise durch Einleiten des Ansatzes in säurehaltiges Wasser und Abtrennen des ausgefallenen Produktes. Soweit die Verfahrensprodukte flüssig sind, und das ist bei einem Grossteil von ihnen der Fall, können sie z.B. durch Destillation, soweit sie fest sind, z.B. durch Destillation oder Kristallisation gereinigt werden.

Die Stoffe der Formel I werden nach dem erfindungsgemässen Verfahren meist in Ausbeuten von 60 bis 80% d.Th. und mehr erhalten, vor allem, wenn als Ausgangsstoffe der Formel III 2-Bromfettsäureester eingesetzt werden. Geht man von den entsprechenden Chlorverbindungen aus, liegen die Ausbeuten häufig niedriger. In beiden Fällen aber ist die Selektivität in bezug auf die Monosubstitution der Verbindungen der Formel II ausgezeichnet.

Die nach dem erfindungsgemässen Verfahren hergestellten 2-(Hydroxiphenoxi)-carbonsäureester sind wertvolle Zwischenprodukte für die Synthese von biologisch, insbesondere herbizid aktiven Wirkstoffen, wie sie z.B. in den folgenden Druckschriften beschrieben sind: DE-OS 2223894, DE-OS 2433067, DE-OS 2546251, DE-OS 2812571, DE-OS 2820032, DE-OS 2914300, DE-OS 2946652, DE-OS 3024265, DE-OS 3004770, EP 2800, EP 3114, EP 23785, EP 24932, EP 29319, US-PS 4236912.

Das erfindungsgemässe Verfahren wird durch die folgenden Beispiele näher erläutert.

*Beispiel 1:*

550 g (4 Mol) Hydrochinon wurden in 2 l wasserfreiem Dimethylsulfoxid vorgelegt und mit 185 g (2,5 Mol) Calciumhydroxid versetzt. Nach einer Rührzeit von 30 min wurde der Ansatz auf 10°C gekühlt. Sodann wurden 362 g (2 mol) 2-Brompropionsäureethylester im Verlaufe von 5 h zugetropft. Nach Beendigung der Zugabe wurde der Ansatz auf Zimmertemperatur erwärmt und 20 h lang nachgerührt und anschliessend in eine Mischung aus 4 l Eiswasser und 500 ml Salzsäure (38%) eingerührt. Das Gemisch wurde mit 3 l Methylenchlorid extrahiert, die Methylenchloridlösung einmal mit Wasser gewaschen.

Es wurden 345 g (1,643 mol) 2-(4-Hydroxiphenoxi)-propionsäureethylester der Formel

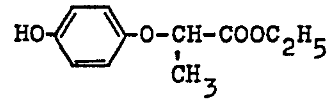

erhalten (Austeute 82,3%). $Kp_{0,2\,mbar}$=133-136°C

*Analyse* für $C_{11}GH_{14}O_4$/M=210

| | | |
|---|---|---|
| Berechnet: | C 62,85 | H 6,66% |
| Gefunden: | C 62,60 | H 6,50% |

*Beispiel 2:*

254 g (2,4 mol) Hydrochinon wurden in 800 ml wasserfreiem Dimethylsulfoxid gelöst und mit 89 g (1,2 mol) Calciumhydroxid versetzt. Nach 30 min langem Rühren bei Zimmertemperatur wurde der Ansatz auf 10°C gekühlt. Unter Aufrechterhaltung dieser Temperatur wurden innerhalb 3 h 268 g (1,2 mol) 2-Brompropionsäure-n-pentyl-ester zugetropft. Dann wurde auf Zimmertemperatur erwärmen gelassen und 20 h lang bei dieser Temperatur gerührt. Schliesslich wurde der Ansatz wie im Beispiel 1 angegeben aufgearbeitet. Es wurden 214 g (0,85 mol) 2-(4-Hydroxiphenoxi)-propionsäure-n-pentylester erhalten (Ausbeute 70,8%). $Kp_{0,2\,mbar}$=149-150°C.

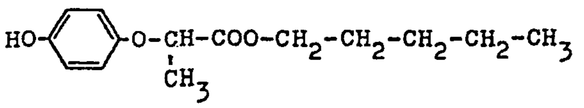

*Analyse* für $C_{14}H_{20}O_4/M=252$

| | | |
|---|---|---|
| Berechnet: | C 66,65 | H 7,99% |
| Gefunden: | C 65,90 | H 7,89% |

*Beispiel 3:*

121 g (1,1 mol) Hydrochinon wurden in 500 ml Dimethylsulfoxid gelöst und die Lösung mit 37 g (0,5 mol) Calciumhydroxid versetzt und 1 h lang bei 45-50°C gerührt. Dann wurden bei derselben Temperatur innerhalb 1 h 82,3 g (0,455 mol) optisch aktiver 2-Chlor-propionsäure-n-butylester ($\alpha_D^{20}=-13,0°$) zugetropft. Der Ansatz wurde weitere 3 h bei 45 bis 50°C gerührt, abgekühlt und wie im Beispiel 1 angegeben aufgearbeitet. Es wurden 49,7 g (0,21 mol) optisch aktiver 2-(4-Hydroxiphenoxi)-propionsäure-n-butylester erhalten (Ausbeute 46,2%). $Kp_{0,22\ mbar}=150-152°C$, $\alpha_D^{20}=+11,8°$.

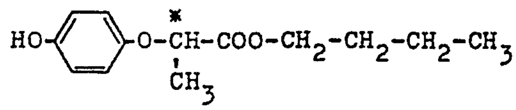

*Analyse* für $C_{13}H_{18}O_4/M=238$

| | | |
|---|---|---|
| Berechnet: | C 65,54 | H 7,56% |
| Gefunden: | C 65,30 | H 7,50% |

Entsprechend den Angaben im Beispiel 1 (einschliesslich der dort genannten Molverhältnisse) wurden die in der folgenden Tabelle zusammengefassten Umsetzungen mit 2-Bromfettsäureestern als Komponenten III durchgeführt.

| Bsp. | Komponente II | Komponente III $R^1$ | $R^2$ | Kp (°C) | Ausbeute (%) |
|---|---|---|---|---|---|
| 4 | Hydrochinon | H | $C_2H_5$ | 140-144 (0,2 mbar) | 48,3 |
| 5 | Hydrochinon | $CH_3$ | $CH_3$ | 127-128 (0,2 mbar) | 79,4 |
| 6 | Hydrochinon | $CH_3$ | $n-C_3H_7$ | 139-142 (0,35 mbar) | 73,1 |
| 7 | Hydrochinon | $CH_3$ | $n-C_4H_9$ | 146-149 (0,2 mbar) | 76,1 |
| 8 | Hydrochinon | $C_2H_5$ | $C_2H_5$ | 138-142 (0,5 mbar) | 68,8 |
| 9 | Resorcin | $CH_3$ | $CH_3$ | 132 (0,2 mbar) | 65,7 |
| 10 | Hydrochinon | $CH_3$ | $n-C_8H_{17}$ | 166-170 (0,25 mbar) | 58 |
| 11 | Hydrochinon | $CH_3$ | $-CH_2-CH=CH_2$ | 136-140 (0,15 mbar) | 62,2 |
| 12 | Hydrochinon | $CH_3$ | $-CH_2-C\equiv CH$ | 148-150 (0,2 mbar) | 65,5 |
| 13 | | $CH_3$ | $-N=\underset{CH_3}{\underset{\mid}{C}}-CH_3$ | | |

$^1$H-NMR-Daten für Beispiel 13
($D_6DMSO$; innerer Standard TMS;
chemische Verschiebung δ in ppm):

$1,83\ (s;\ -N=\underset{CH_3}{\underset{\mid}{C}}-CH_3)$, $1,90\ (s;\ -N=\underset{CH_3}{\underset{\mid}{C}}-CH_3)$,

$1,50\ (d;\ R^1=CH_3)$, $4,88\ (q;\ -\underset{\mid}{\overset{\mid}{C}}H)$.

$D_6DMSO$ = deuteriertes Dimethylsulfoxid
TMS = Tetramethylsilan.

## Patentansprüche

1. Verfahren zur Herstellung von 2-(Hydroxiphenoxi)-carbonsäureestern der allgemeinen Formel

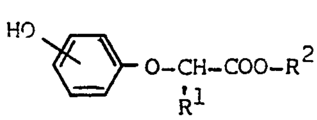 I,

in der $R^1$ Wasserstoff oder eine niedermolekulare Alkylgruppe und $R^2$ einen gesättigten oder ungesättigten Kohlenwasserstoffrest mit bis zu 10 Kohlenstoffatomen, der gegebenenfalls mit Phenoxi- oder Cyangruppen, Halogen oder Trialkylsilyl substituiert ist, oder einen Rest der Formel 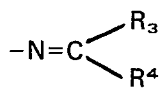 und $R^3$ und $R^4$ gleiche oder verschiedene Kohlenwasserstoffreste mit zusammen bis zu 9 Kohlenstoffatomen, die auch miteinander verbunden sein können, darstellen, durch Umsetzung eines Dihydroxibenzols mit einem 2-Halogenfettsäureester in organischer Lösung in Gegenwart einer Base, dadurch gekennzeichnet, dass man eine Verbindung der Formel

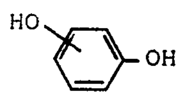 II

mit einem 2-Halogenfettsäureester der Formel

$$X-\underset{R^1}{\underset{\mid}{C}}H-COO-R^2 \qquad III$$

in der $R^1$ und $R^2$ die oben angegebene Bedeutung haben und X ein Chlor- oder Bromatom bedeutet, in Gegenwart von Calciumhydroxid als Base in Dimethylsulfoxid bei einer Temperatur zwischen 0 und +100°C umsetzt, wobei das Molverhältnis der Ausgangsstoffe II:III zu Beginn der Umsetzung wenigstens 1,5:1 beträgt.

2. Verfahren nach Anspruch 1, bei dem ein Ausgangsstoff III eingesetzt wird, dessen Rest $R^1$ Wasserstoff, Methyl oder Ethyl ist.

3. Verfahren nach Anspruch 1, bei dem ein Ausgangsstoff III eingesetzt wird, dessen Rest $R^1$ Alkyl und der optisch aktiv ist.

## Claims

1. A process for the preparation of a 2-(hydroxyphenoxy)-carboxylate of the general formula

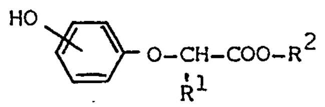 I,

where $R^1$ is hydrogen or a low molecular weight alkyl group, and $R^2$ is a saturated or unsaturated hydrocarbon radical of up to 10 carbon atoms which is unsubstituted or substituted by phenoxy, cyano, halogen or trialkylsilyl, or is a radical of the formula

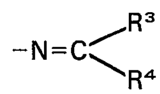

where $R^3$ and $R^4$ are identical or different hydrocarbon radicals which are of up to 9 carbon atoms together and may also be bonded to one another, by reacting a dihydroxybenzene with a 2-halo-fatty acid ester in solution in an organic solvent in the presence of a base, wherein a compound of the formula

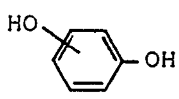 II

is reacted with a 2-halo-fatty acid ester of the formula

$$X-\underset{\substack{| \\ R^1}}{CH}-COO-R^2 \qquad III$$

where $R^1$ and $R^2$ have the above meanings and X is chlorine or bromine, in the presence of calcium hydroxide as the base, in dimethylsulfoxide, at from 0 to +100°C, the molar ratio of starting material II to starting material III at the beginning of the reaction being at least 1,5:1.

2. A process as claimed in claim 1, wherein a starting material III is employed in which the radical $R^1$ is hydrogen, methyl or ethyl.

3. A process as claimed in claim 1, wherein a starting material III is employed which is optically active and in which the radical $R^1$ is alkyl.

## Revendications

1. Procédé de préparation d'esters de l'acide (hydroxy-phénoxy)-2-carboxylique de la formule générale

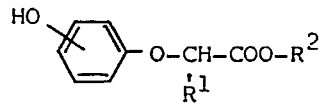 (I),

dans laquelle $R^1$ désigne un atome d'hydrogène ou un radical alcoyle à bas poids molécuaire et $R^2$ un groupe hydrocarboné saturé ou non avec jusqu'à 10 atomes de carbone, éventuellement substitué par des groupes phénoxy ou cyano, halogène ou trialcoyl-silyle, ou un groupe de la formule

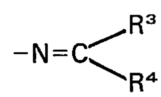

où $R^3$ et $R^4$ désignent des groupes hydrocarbonés identiques ou différents, comportant au total jusqu'à 9 atomes de carbone et éventuellement reliés l'un à l'autre, par réaction d'un dihydroxy-benzène en solution organique et en présence d'une base avec un ester d'un acide gras halogéné en position 2, caractérisé en ce que l'on effectue la réaction d'un composé de la formule

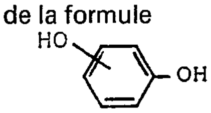 (II)

et d'un ester d'acide gras halogéné en position 2, de la formule

$$X-\underset{\substack{| \\ R^1}}{CH}-COO-R^2 \qquad (III)$$

dans laquelle $R^1$ et $R^2$ possèdent la signification définie et X désigne un atome de chlore ou de brome, dans du sulfoxyde de diméthyle, entre 0 et +100°C et en présence d'hydroxyde de calcium comme base, le rapport des proportions molaires des composés de départ II et III étant au moins égal à 1,5:1 au début de la réaction.

2. Procédé suivant la revendication 1, employant comme composé de départ III un composé, dont $R^1$ représente un atome d'hydrogène ou un radical méthyle ou éthyle.

3. Procédé suivant la revendication 1, employant comme composé de départ III un composé, dont $R^1$ désigne un radical alcoyle et qui est optiquement actif.